# EUROPÄISCHE PATENTSCHRIFT

(11) **EP 2 382 175 B1**
(45) Veröffentlichungstag und Bekanntmachung des Hinweises auf die Patenterteilung: **25.02.2015**
(21) Anmeldenummer: 09797041.2
(22) Anmeldetag: 21.12.2009
(51) Int. Cl.: C07C 45/80, C07C 47/127

(54) **VERFAHREN ZUR REINIGUNG WÄSSRIGER GLYOXAL-LÖSUNGEN**
PROCESS FOR PURIFYING AQUEOUS GLYOXAL SOLUTIONS
PROCÉDÉ DE PURIFICATION DE SOLUTIONS AQUEUSES DE GLYOXAL

(30) Priorität: 29.12.2008 EP 08173007
(43) Veröffentlichungstag der Anmeldung: 02.11.2011
(73) Patentinhaber: BASF SE, 67056 Ludwigshafen (DE)
(72) Erfinder: DECKERT, Petra, 69245 Bammental (DE); GROLL, Peter, 67125 Dannstadt-Schauernheim (DE); RUMPF, Bernd, 68766 Hockenheim (DE); HORN, Christian, 67308 Rüssingen (DE)
(86) Internationale Anmeldenummer: PCT/EP2009/067611
(87) Internationale Veröffentlichungsnummer: WO 2010/076252

(56) Entgegenhaltungen:
- EP-A2- 0 219 045
- DE-A1- 3 402 733
- US-A- 3 860 656
- J. H. MCCAIN: "Deacidification of glyoxal by liquid ion exchange" IND. ENG. CHEM. PROCESS DES. DEV., Bd. 19, 1980, Seiten 494-497, XP002588023

## Beschreibung

Die vorliegende Erfindung betrifft ein Verfahren zur Reinigung von wässrigen Glyoxal-Lösungen, die mindestens eine Säure enthalten, mit einer lonentauscher-Lösung sowie die nach diesem Verfahren erhältlichen wässrigen Glyoxal-Lösungen.

Glyoxal wird beispielsweise als Hilfskomponente in der Textil- oder Papierindustrie verwendet. Glyoxal wird üblicherweise durch Oxidation von Acetaldehyd oder durch Oxidehydrierung des entsprechenden Glykols an einem katalytischen Festbett hergestellt. Als Katalysator kommt dabei beispielsweise mit Phosphor dotiertes Kupfer zum Einsatz. Die bei diesem Verfahren anfallenden wässrigen Glyoxal-Lösungen weisen dabei Nebenprodukte auf, die vor ihrer weiteren Verwendung abgetrennt werden müssen. Typische Nebenprodukte sind dabei Formaldehyd, Glykolaldehyd, Ameisensäure, Essigsäure, sowie schwerflüchtige Säuren wie Glyoxylsäure, Glykolsäure und Oxalsäure. Das durch Oxidehydrierung aus Glykol hergestellte Glyoxal enthält in der Regel nicht mehr als 2 Gew.-% Säure. Für den kommerziellen Einsatz des Glyoxals werden jedoch Säurezahlen von < 5 mg KOH/g gefordert.

Zur Abtrennung der oben genannten Säuren, vor allem der schwerflüchtigen Säuren, sind im Stand der Technik verschiedene Verfahren beschrieben. US 3 270 062 beschreibt ein Verfahren zur Reinigung von wässrigen Glyoxal-Lösungen durch Behandlung der Glyoxal-Lösung mit einem festen Ionenaustauscher. Dieses Verfahren hat den Nachteil einer diskontinuierlichen Arbeitsweise. Außerdem müssen die Ionentauscher wegen der hohen Säurewerte der eingesetzten wässrigen Glyoxal-Lösungen häufig regeneriert werden. Aus diesem Grund fallen bei diesem Verfahren erhebliche Mengen verdünnter Glyoxal-Lösungen an, so dass das in US 3 270 062 beschriebene Verfahren nicht wirtschaftlich betrieben werden kann.

US 3,860,656 beschreibt ein Verfahren zur Entfernung von sauren Verunreinigungen aus wässrigen Glyoxal-Lösungen durch Behandlung der Glyoxal-Lösung mit einer Lösung hochmolekularer tertiärer Amine oder quartärnerer Amoniumsalze in Bicarbonatform in einem organischen Lösungsmittel. Bei diesem Verfahren führt man die beiden Lösungen in einer vielstufigen Extraktionskolonne zueinander im Gegenstrom. Dadurch wird zwar eine kontinuierliche Reinigung erreicht, es sind jedoch lange Verweilzeiten erforderlich. Außerdem ist bei diesem Verfahren zur Vermeidung von zu großen Gly-oxal-Verlusten eine Reextraktion der organischen Phase mit Wasser notwendig. Dies führt dazu, dass dieses Verfahren unattraktiv ist.

Eine Aufarbeitung des durch Oxidation von Acetaldehyd oder durch Oxidehydrierung hergestellten Glyoxals ist zwingend notwendig, da die nach diesem Verfahren erhaltenen Glyoxal-Lösungen eine starke Gelbfärbung aufweisen. Für die kommerziell eingesetzten Glyoxal-Lösungen werden im Endprodukt typischerweise Farbzahlen von < 200 Apha gefordert.

DE 34 02 733 beschreibt ein Verfahren zur Reinigung wässriger Glyoxal-Lösungen durch Extraktion der in der Glyoxal-Lösung enthaltenen Säuren (Säureextraktion) mit einer Lösung eines tertiären Amins mit einem organischen Lösungsmittel in einem Rührgefäß.

Bei dem dort beschriebenen Verfahren werden wässrige Glyoxal-Lösungen erhalten, die eine Säurezahl < 5 mg KOH/g und eine niedrige Farbzahl aufweisen. Die nach diesem Verfahren erhältlichen Glyoxal-Lösungen entsprechen somit denen, die für die kommerziell eingesetzten Glyoxal-Lösungen gefordert werden. Bei dem in DE 34 02 733 beschriebenen Verfahren ist zudem der Glyoxal-Verlust in die Lösung aus tertiärem Amin und organischem Lösungsmittel gering. Die in diesem Verfahren erhaltenen Glyoxal-Lösungen haben jedoch eine nicht zufriedenstellende Lagerstabilität. So kann bei der Lagerung der Glyoxal-Lösungen eine fortschreitende Zunahme der Farbzahl beobachtet werden. Die nach dem Verfahren hergestellten Glyoxal-Lösungen weisen somit schnell Farbzahl-Werte auf, die außerhalb der für den kommerziellen Einsatz geforderten Spezifikationen liegen.

Aufgabe der vorliegenden Erfindung ist es, ein Verfahren zur Reinigung von wässrigen Glyoxal-Lösungen bereitzustellen, das die vorstehend beschriebenen Nachteile überwindet und wässrige Glyoxal-Lösungen liefert, die niedrige Säure- und Farbzahlen sowie eine verbesserte Lagerstabilität aufweisen.

Es wurde nun überraschend gefunden, dass eine wässrige Glyoxal-Lösung mit niedriger Säure und Farbzahl sowie verbesserter Lagerstabilität erhalten werden kann, wenn die zur Säureextraktion verwendeten Extraktionsmittel möglichst vollständig abgetrennt werden.

Gegenstand der Erfindung ist somit ein Verfahren zur Reinigung einer wässrigen Gly-oxal-Lösung, die mindestens eine Säure enthält, durch extraktive Säureabtrennung, umfassend die Schritte
I) Vermischen einer wässrigen Glyoxal-Lösung mit einer Ionentauscher-Lösung, enthaltend 20 bis 60 Gew.-% eines geradkettigen oder verzweigten aliphatischen tertiären Amins mit einem Molekulargewicht von 300 bis 600 g/mol und 80 bis 40 Gew.-% eines oder mehrerer mit Wasser nicht beliebig mischbaren organischen Lösungsmittels, ausgewählt aus aliphatischen Alkoholen mit 3 bis 15 C-Atomen bei einer Verweilzeit von < 5 Minuten und einer Temperatur von 30 bis 100°C, zur Herstellung einer Mischung,
II) Auftrennung der Mischung aus Schritt I) in eine extrahierte wässrige Gly-oxal-Lösung und eine beladene lonentauscher-Lösung und Abtrennung der beladenen lonentauscher-Lösung bei Temperaturen von 30 bis 100°C, und
III) Auftrennung der extrahierten wässrigen Glyoxal-Lösung in tertiäres Amin und gereinigte wässrige Glyoxal-Lösung und Abtrennung des tertiären Amins bei Temperaturen < 30°C.

Die nach dem erfindungsgemäßen Verfahren erhältlichen wässrigen Glyoxal-Lösungen zeichnen sich durch niedrige Säure- und Farbzahlen sowie durch eine deutlich verbesserte Lagerstabilität aus.

Zur Entfernung weiterer Nebenprodukte aus wässriger Glyoxal-Lösung werden im Anschluss an die Säureextraktion häufig Adsorber eingesetzt. Es hat sich gezeigt, dass Reste der lonentauscher-Lösung die zur Abtrennung weiterer Nebenprodukte eingesetzten Adsorber belegen und somit deren Standzeit verkürzen. Glyoxal-Lösungen, die nach dem erfindungsgemäßen Verfahren gereinigt wurden, weisen einen Gehalt an tertiären Aminen auf, der gegenüber den nach dem Stand der Technik erhaltenen wässrigen Glyoxal-Lösungen deutlich reduziert ist. Dadurch wird die weitere Aufarbeitung der nach dem erfindungsgemäßen Verfahren erhältlichen wässrigen Glyoxal-Lösungen deutlich vereinfacht, und die Standzeiten der eingesetzten Adsorber werden deutlich verlängert.

### Schritt I)

In Schritt I) des erfindungsgemäßen Verfahrens werden wässrige Glyoxal-Lösungen, die herstellungsbedingt mindestens eine Säure als Nebenprodukt enthalten, mit einer lonentauscher-Lösung vermischt. In Schritt I) des erfindungsgemäßen Verfahrens können alle im Stand der Technik bekannten wässrigen Glyoxal-Lösungen eingesetzt werden, die herstellungsbedingt mindestens eine Säure als Nebenprodukt enthalten. Üblicherweise werden wässrige Glyoxal-Lösungen, die durch Oxidation von Acetaldehyd oder durch Oxidehydrierung von Monoethylenglykol an einem Katalysator erhalten werden, eingesetzt. Besonders bevorzugt sind wässrige Glyoxal-Lösungen, die entsprechend dem in DE 19 23 048 beschriebenen Verfahren durch Oxidehydrierung an einem Katalysatorfestbett, enthaltend Phosphor dotiertes Kupfer als Katalysator, zugänglich sind.

Die wässrigen Glyoxal-Lösungen können auch nach den in DE 500 04 079 und EP 11 69 119 beschriebenen Verfahren erhalten werden. Dazu wird Monoethylenglykol in einem geeigneten Verdampfer in die Gasphase überführt. Zur Oxidehydrierung des Monoethylenglykols wird Sauerstoff bereitgestellt und das Eduktgemisch wird einem Reaktor zugeführt. Das den Reaktor verlassende gasförmige Produktgemisch, welches neben Glyoxal andere Nebenprodukte enthält, wird abgekühlt. Dabei werden die kondensierbaren Komponenten, wie Wasser und Glyoxal, sowie Nebenprodukte wie Form-aldehyd, Glykolaldehyd, Ameisensäure, sowie schwerflüchtige Säuren wie Glyoxylsäure, Glykolsäure und Oxalsäure, auskondensiert. Die weitere Aufarbeitung des Produktstroms erfolgt durch Abstrippung leicht siedender Komponenten, wie Formaldehyd, Ameisensäure und Essigsäure mittels Wasserdampf. Die so erhaltenen wässrigen Glyoxal-Lösungen enthalten noch schwerflüchtige Säuren wie Glyoxylsäure, Glykolsäure und Oxalsäure. Auch die nach den Verfahren gemäß DE 500 04 079 und EP 11 69 119 zugänglichen Glyoxal-Lösungen sind zum Einsatz in das erfindungsgemäße Verfahren besonders geeignet.

Die in das erfindungsgemäße Verfahren in Schritt I) eingesetzten wässrigen Glyoxal-Lösungen enthalten also mindestens eine Säure, bevorzugt ausgewählt aus der Gruppe bestehend aus Ameisensäure, Essigsäure, Glyoxylsäure, Glykolsäure und Oxalsäure oder Mischungen aus zwei oder mehr der vorstehend genannten Säuren, in einer Konzentration im Bereich von 0,2 bis 2 Gew.-%, bezogen auf das Gesamtgewicht der wässrigen Glyoxal-Lösung.

Die Säurezahlen der in das erfindungsgemäße Verfahren eingesetzten wässrigen Glyoxal-Lösungen können in weiten Bereichen variieren. Bevorzugt sind jedoch wässrige Glyoxal-Lösungen, die Säurezahlen ≤ 100 mg KOH/g, bevorzugt ≤ 50 mg KOH/g und besonders bevorzugt ≤ 30 mg KOH/g aufweisen.

Auch der Glyoxalgehalt der in das erfindungsgemäße Verfahren eingesetzten wässrigen Glyoxal-Lösungen ist unkritisch und kann in weiten Bereichen variiert werden. Bevorzugt werden jedoch wässrige Glyoxal-Lösungen mit einem Glyoxalgehalt ≤ 70 Gew.-%, bevorzugt ≤ 55 Gew.-% und besonders bevorzugt ≤ 45 Gew.-% eingesetzt, wobei Gew.-% jeweils bezogen auf das Gesamtgewicht der eingesetzten wässrigen Glyoxal-Lösung bezogen sind.

Die wässrige Glyoxal-Lösung wird in Schritt I) mit einer lonentauscher-Lösung vermischt. Unter lonentauscher-Lösung im Rahmen der vorliegenden Erfindung werden Mischungen, enthaltend ein tertiäres Amin und ein mit Wasser nicht beliebig mischbares organisches Lösungsmittel, wie oben definiert verstanden. Bevorzugte tertiäre Amine sind Trioctylamin, Trinonylamin, Tridecylamin und Tridodecylamin. Es ist auch möglich, Mischungen aus zwei oder mehr der bevorzugten tertiären Amine einzusetzen. Darüber hinaus können auch ein oder mehrere der bevorzugten tertiären Amine als Mischung mit einem oder mehreren weiteren Aminen eingesetzt werden.

Der Begriff tertiäres Amin im Rahmen der vorliegenden Erfindung umfasst somit auch Mischungen von einem tertiären Amin mit einem oder mehreren weiteren Aminen. Besonders bevorzugt ist der Einsatz von binären Mischungen aus zwei tertiären Aminen ausgewählt aus Trioctylamin, Trinonylamin, Tridecylamin und Tridodecylamin. Insbesondere bevorzugt sind Mischungen aus Trioctylamin und Tridecylamin.

Mit Wasser nicht beliebig mischbare organische Lösungsmittel sind bevorzugt aliphatische Alkohole mit 8 bis 13 C-Atomen. Insbesondere bevorzugt sind Pentanol, Hexanol, 2-Ethylhexanol, Octanol, Decanol und Isodecanol. Als mit Wasser nicht beliebig mischbare organische Lösungsmittel können auch Mischungen der vorstehend genannten Lösungsmittel eingesetzt werden. Der Begriff "mit Wasser nicht beliebig mischbares organisches Lösungsmittel" im Rahmen der vorliegenden Erfindung umfasst somit auch Mischungen von 2 oder mehr der vorstehend genannten Lösungsmittel.

Die lonentauscher-Lösung enthält im Allgemeinen 20 bis 60 Gew.-%, bevorzugt 30 bis 50 Gew.-% eines tertiären Amins und 80 bis 40 Gew.-% bevorzugt 70 bis 50 Gew.-% eines mit Wasser nicht beliebig mischbaren organischen Lösungsmittels, jeweils bezogen auf das Gesamtgewicht der lonentauscher-Lösung.

In Schritt I) des erfindungsgemäßen Verfahrens wird die wässrige Glyoxal-Lösung mit der lonentauscher-Lösung vermischt. Die Vermischung der wässrigen Glyoxal-Lösung mit der lonentauscher-Lösung erfolgt dabei bei Temperaturen von 30 bis 100°C, bevorzugt von 30 bis 80°C und besonders bevorzugt von 30 bis 60°C. Das Volumenverhältnis von wässriger Glyoxal-Lösung und lonentauscher-Lösung ist frei wählbar. Bevorzugt sind Volumenverhältnisse zwischen 10:1 und 1:3.

Das Vermischen der wässrigen Glyoxal-Lösung mit der lonentauscher-Lösung kann dabei in jedem bekannten Mischapparat erfolgen. Es hat sich gezeigt, dass die Verweilzeit der beiden Lösungen möglichst kurz sein sollte, so dass sich das Phasengleichgewicht von Glyoxal in der Phase der lonentauscher-Lösung nicht einstellt. Unter Verweilzeit wird im Rahmen der vorliegenden Erfindung die Zeitspanne verstanden, während der sich die wässrige Glyoxal-Lösung und die lonentauscher-Lösung im Mischapparat unter Durchmischung befinden. Dadurch kann der Übergang von Glyoxal aus der wässrigen Glyoxal-Lösung in die lonentauscher-Lösung und damit der Verlust von Glyoxal, gering gehalten werden. Geeignete Verweilzeiten liegen unter 5 Minuten, bevorzugt im Bereich von 1 Sekunde bis 2 Minuten, insbesondere bevorzugt im Bereich von 1 bis 20 Sekunden.

Dabei werden die in der wässrigen Glyoxal-Lösung enthaltenen Säuren an das in der Ionentauscher-Lösung enthaltenden tertiären Amin gebunden. Dabei entsteht eine beladene lonentauscher-Lösung, die in der Glyoxal-Lösung enthaltene Säuren enthält. Geeignete Mischapparate ermöglichen eine möglichst rückvermischungsfreie Dispergierung bei sehr kurzer Verweilzeit, wobei gleichzeitig eine gute Phasentrennung (Schritt II)) ermöglicht wird. Die Vermischung der beiden Lösungen erfolgt dabei bevorzugt einstufig durch ein gleichzeitiges Einleiten in einen Mischapparat. In einer bevorzugten Ausführungsform wird hierzu ein statischer Mischer eingesetzt. In einer weiteren bevorzugten Ausführungsform wird ein In-Line-Mischer oder ein Lochblenden-Mischer eingesetzt.

### Schritt II)

Nach Vermischen der beiden Lösungen erfolgt die Auftrennung der Mischung aus Schritt I) durch Phasentrennung in eine wässrige Glyoxal-Lösung, aus der die Säure extrahiert wurde (extrahierte wässrige Glyoxal-Lösung) und eine beladene lonentauscher-Lösung. Die beladene lonentauscher-Lösung kann durch Behandlung mit alkalisch wirkenden Mitteln wie Natriumhydroxid, Kaliumhydroxid oder Natriumcarbonat regeneriert werden. Die in Schritt I) erhaltene Mischung wird in einer Ausführungsform einer Trennapparatur zugeführt, in der die Auftrennung und Abtrennung der beladenen lonentauscher-Lösung aus Schritt I) von der extrahierten wässrigen Glyoxal-Lösung durchgeführt wird. Die Auftrennung und Abtrennung kann in jeder beliebigen Trennapparatur, wie Phasenscheider, Zentrifuge oder Phasenscheider in Kombination mit Koaleszierfilter erfolgen.

Der Koaleszierfilter kann dabei dem Phasenscheider vorgeschaltet oder im Phasenscheider integriert sein. Geeignete Materialien für die Koaleszierfilter sind alle Materialien, die gegenüber der lonentauscher-Lösung und der wässrigen Glyoxal-Lösung, sowie gegenüber der beladenen lonentauscher-Lösung und der extrahierten wässrigen Glyoxal-Lösung, unter den Betriebsbedingungen in Schritt II) beständig sind und geeignete Benetzungseigenschaften aufweisen. Geeignete Materialien für einen Koaleszierfilter sind beispielsweise Acrylfasern, die mit Phenolharz gebunden sind. Der Phasenscheider kann als Trennhilfen Einbauten wie Gestricke, Füllkörper und/oder Platten enthalten.

Die Auftrennung der Mischung aus Schritt I) in die extrahierte wässrige Glyoxal-Lösung und die beladene lonentauscher-Lösung wird dabei bei Temperaturen von 30 bis 100°C, bevorzugt bei 30 bis 80°C und besonders bevorzugt bei 30 bis 60°C vorgenommen.

In einer bevorzugten Ausführungsform des erfindungsgemäßen Verfahrens werden die Schritte I) und II) kontinuierlich nacheinander durchgeführt. Dazu wird die wässrige Glyoxal-Lösung zunächst in einem der unter Schritt I) beschriebenen Mischapparate mit der lonentauscher-Lösung gemischt und anschließend direkt einem der vorstehend beschriebenen Trennapparaturen zugeführt und in extrahierte wässrige Glyoxal-Lösungen und beladene lonentauscher-Lösung aufgetrennt.

In einer weiteren bevorzugten Ausführungsform werden Schritt I) und Schritt II) in einer einzigen Apparatur durchgeführt, d.h. Vermischen und Auf- und Abtrennung erfolgen in derselben Apparatur. Hierfür sind beispielsweise Zentrifugalextraktoren von CINC oder Podbielniak (POD) geeignet.

In einer insbesondere bevorzugten Ausführungsform wird Schritt I) in einem statischen Mischer und Schritt II) in einer Zentrifuge durchgeführt. Es ist jedoch auch möglich, Schritt I) in einem statischen Mischer und Schritt II) in einem Phasenabscheider allein oder in einem Phasenscheider in Kombination mit einem Koaleszierfilter durchzuführen.

### Schritt III)

In Schritt III) wird die in Schritt II) erhaltene extrahierte wässrige Glyoxal-Lösung einer weiteren Abtrennung von tertiärem Amin aus der lonentauscher-Lösung unterzogen. Es hat sich überraschenderweise gezeigt, dass die vorstehend beschriebene Abtrennung der Ionentauscher-Lösung (Schritt II)) nicht vollständig erfolgt und dass geringe Mengen an tertiärem Amin und des Lösungsmittels aus der lonentauscher-Lösung in der extrahierten wässrigen Glyoxal-Lösung verbleiben. Typischerweise weisen die extrahierten Glyoxal-Lösungen tertiäre Amine und Lösungsmittel aus der lonentauscher-Lösung von bis zu 1000 ppm auf. Es wurden auch Mengen im Bereich von 5 bis 500 ppm und 10 bis 250 ppm gemessen, jeweils bezogen auf das Gesamtgewicht der extrahierten wässrigen Glyoxal-Lösung.

Die Auftrennung der extrahierten wässrigen Glyoxal-Lösung in eine vom tertiären Amin weitestgehend befreite wässrige Glyoxal-Lösung (gereinigte wässrige Glyoxal-Lösung) und tertiäres Amin erfolgt durch Phasentrennung

Zur Abtrennung des tertiären Amins wird die extrahierte wässrige Glyoxal-Lösung aus Schritt II) auf Temperaturen < 30°C, bevorzugt auf Temperaturen im Bereich von 0 bis < 30°C und insbesondere bevorzugt auf Temperaturen im Bereich von 10 bis 25°C abgekühlt. Dadurch kommt es zu einer Abscheidung von tertiärem Amin aus der extrahierten wässrigen Glyoxal-Lösung aus Schritt II). Neben der Abscheidung von tertiärem Amin kann es auch zu einer Abscheidung des mit Wasser nicht beliebig mischbaren organischen Lösungsmittels kommen.

Die Abtrennung des abgeschiedenen tertiären Amins kann durch jedes bekannte Trennverfahren erfolgen. Geeignete Trennverfahren wurden vorstehend (Schritt II)) beschrieben. Bevorzugt ist die Abtrennung durch einen Koaleszierfilter mit anschließendem Phasenscheider oder durch einen Phasenscheider mit integriertem Koaleszierfilter oder durch eine Zentrifuge.

Insbesondere bevorzugt wird Schritt III) mit einem Koaleszierfilter mit anschließendem Phasenscheider oder mit einem Phasenscheider mit integriertem Koaleszierfilter durchgeführt. Bevorzugte Materialien für den Koaleszierfilter in Schritt III) sind Kunststoffe, insbesondere Polypropylen und Polyethylen oder Mischungen daraus.

Es wurde überraschenderweise gefunden, dass die Reinigung von wässrigen Glyoxal-Lösungen nach dem erfindungsgemäßen Verfahren zu einer nahezu vollständigen Abtrennung der lonentauscher-Lösung und damit zu einer Abtrennung des darin enthaltenen tertiären Amins aus der wässrigen Glyoxal-Lösung führt. Die nach dem erfindungsgemäßen Verfahren hergestellten wässrigen Glyoxal-Lösungen weisen einen Gehalt an tertiären Aminen (bezogen auf das Gesamtgewicht der wässrigen Glyoxal-Lösung) von weniger als 100 ppm, bevorzugt weniger als 50 ppm und insbesondere bevorzugt von weniger als 20 ppm auf. Die Abtrennung der lonentauscher-Lösung und damit die Abtrennung des darin enthaltenen tertiären Amins führen zu einer Verbesserung der Lagerstabilität der wässrigen Glyoxal-Lösung.

Die Erfindung wird durch die Beispiele näher erläutert, ohne sie darauf zu beschränken.

### Beispiele:

### Beispiel 1: Farbbildung in der lonentauscher-Lösung

In Schüttelflaschen wurde lonentauscher-Lösung, bestehend aus 40% Hostarex A327 (Mischung aus 50% Tridodecylamin und 50% Trioctylamin von der Firma Clariant) und 60% Isodekanol, über einen Zeitraum von einer Stunde bei 60°C temperiert und die Farbbildung in der lonentauscher-Lösung optisch bewertet. Es wurden Ansätze der reinen lonentauscher-Lösung ohne Zusatz untersucht sowie Flaschen, in denen geringe Mengen Glyoxal und/oder Säure (hier Ameisensäure) hinzu gegeben wurden.

**Tabelle 1**

| **Ionentauscher**-**Lösung** | **Farbbildung in der Ionentauscher**-**Lösung** |
|---|---|
| ohne Zugabe | - |
| Zugabe von Säure | - |
| Zugabe von Glyoxal | + |
| Zugabe von Säure + Glyoxal | ++ |

| | |
|---|---|
| - keine Verfärbung + schwache Verfärbung ++ starke Verfärbung | |

In der reinen lonentauscher-Lösung, d.h. ohne Zugabe und bei ausschließlicher Zugabe von Säure, wird bei Verweilzeit und Temperierung keine Farbbildung in der lonentauscher-Lösung beobachtet. Bei Zugabe von Glyoxal wird eine Verfärbung beobachtet, die stark zunimmt, wenn zusätzlich zum Glyoxal Säure in die lonentauscher-Lösung eingelöst wird.

### Beispiel 2: Farbbildung im Alkohol und in der Aminmischung

In Schüttelflaschen wurde die Farbbildung in den beiden Bestandteilen der lonentauscher-Lösung, d.h. der Aminmischung Hostarex (Mischung aus Tridodecylamin und Trioctylamin) und des Alkohols Isodekanol getrennt voneinander untersucht. Es wurden Ansätze der Aminmischung bzw. des Alkohols untersucht, in denen geringe Mengen Glyoxal sowie Glyoxal und Säure (hier Ameisensäure) hinzu gegeben wurden. Die Schüttelproben wurden über einen Zeitraum von 1 Stunde bei 60°C temperiert und die Farbbildung in der Aminmischung sowie im Alkohol wurden optisch bewertet.

**Tabelle 2**

| **Ionentauscher**-**Lösung** | **Farbbildung in Alkohol** | **Farbbildung in Aminmischung** |
|---|---|---|
| Zugabe von Glyoxal | - | + |
| Zugabe von Säure + Glyoxal | - | ++ |

| | | |
|---|---|---|
| - keine Verfärbung + schwache Verfärbung ++ starke Verfärbung | | |

In dem Alkohol kam es weder bei der Zugabe von Glyoxal noch bei der Zugabe von Glyoxal und Säure zu einer Farbbildung. In der Aminmischung wurde Farbbildung allein schon nach der Zugabe von Glyoxal beobachtet. Die Farbbildung verstärkt sich, wenn zusätzlich zum Glyoxal noch Säure in die Aminmischung eingelöst wird.

### Beispiel 3: Farbzahlen in der Glyoxal-Lösung abhängig von der Temperatur und der Menge der lonentauscher-Lösung

Es wurden Schüttelversuche mit Glyoxal-Lösungen aus dem Produktionsprozess durchgeführt. Im Vergleich dazu wurden Schüttelversuche mit derselben Glyoxal-Lösung durchgeführt, der 0,1 kg lonentauscher-Lösung, bezogen auf 1 kg Glyoxal-Lösung, zugesetzt wurden.

Es wurden Proben bei 20, 40 und 60°C jeweils für 1 Stunde temperiert. In den klaren Glyoxal-Lösungen wurden die Farbzahlen nach der Temperierung nach 24 Stunden bestimmt. Die gemessenen Farbzahlen ohne die Zugabe von lonentauscher-Lösung zeigen, dass überraschenderweise die Farbzahl mit steigender Temperatur der Temperierung ansteigen. Dies kann durch das Vorhandensein von gelöster lonentauscher-Lösung erklärt werden.

Ist lonentauscher-Lösung als zweite Phase anwesend, sind die gemessenen Farbzahlen in der Glyoxal-Lösung erstaunlicherweise deutlich höher, als wenn nur Glyoxal-Lösung in der Schüttelflasche vorhanden ist. Es bildet sich in der lonentauscher-Lösung Farbe, welche zum Teil in die Glyoxal-Lösung übergeht. Die Menge der gebildeten Farbe hängt von der Temperatur ab. Sie ist umso größer, je höher die Temperatur ist.

**Tabelle 3**

| **reine Glyoxalprobe** | **gemessene Apha-Farbzahl in Apha nach 24 h*** | **gemessene Apha-Farbzahl in Apha nach 24 h*** | **gemessene Apha-Farbzahl in Apha nach 24 h*** |
|---|---|---|---|
| | **20°C** | **40°C** | **60°C** |
| ohne lonentauscher-Lösung | 20 | 23 | 39 |
| mit lonentauscher-Lösung | 57 | 81 | 208 |

| | | | |
|---|---|---|---|
| *gemessen wurde die Glyoxal-Lösung nach vollständiger Phasentrennung | | | |

### Beispiel 4: Farbzahlen in der Glyoxal-Lösung vor und nach Schritt III

In einer Produktionsanlage wurden zeitgleich zwei Proben der Glyoxal-Lösung entnommen. Probe 1 wurde vor und Probe 2 nach der Abtrennung von Amin aus der Glyoxal-Lösung gemäß Schritt III entnommen. Die Abtrennung von Amin aus der Glyoxal-Lösung erfolgt in der Produktionsanlage gemäß Schritt III des erfindungsgemäßen Verfahrens durch Abkühlen und Trennung der beiden Phasen mit Hilfe eines Koaleszierfilters.

Die Proben wurden bei Raumtemperatur ca. 20 Tage gelagert. In den klaren Glyoxal-Lösungen wurde die Farbzahl nach Apha bestimmt.

In Probe 1 wurde eine Farbzahl von 204 Apha gemessen, in Probe 2 wurde eine Farbzahl von 169 Apha gemessen.

## Patentansprüche

1. Verfahren zur Reinigung einer wässrigen Glyoxal-Lösung, die mindestens eine Säure enthält, durch extraktive Säureabtrennung umfassend die Schritte
I) Vermischen einer wässrigen Glyoxal-Lösung mit einer Ionentauscher-Lösung, enthaltend 20 bis 60 Gew.-% eines geradkettigen oder verzweigten aliphatischen tertiären Amins mit einem Molekulargewicht von 300 bis 600 g/mol und 80 bis 40 Gew.-% eines oder mehrerer mit Wasser nicht beliebig mischbaren organischen Lösungsmittels, ausgewählt aus aliphatischen Alkoholen mit 3 bis 15 C-Atomen bei einer Verweilzeit von < 5 Minuten und einer Temperatur von 30 bis 100°C, zur Herstellung einer Mischung,
II) Auftrennung der Mischung aus Schritt I) in eine extrahierte wässrige Glyoxal-Lösung und eine beladene Ionentauscher-Lösung und Abtrennen der beladenen Ionentauscher-Lösung bei Temperaturen von 30 bis 100°C und
III) Auftrennung der extrahierten wässrigen Glyoxal-Lösung in tertiäres Amin und gereinigte wässrige Glyoxal-Lösung und Abtrennung des tertiären Amins bei Temperaturen < 30°C.

2. Verfahren gemäß Anspruch 1, **dadurch gekennzeichnet, dass** die Auftrennung in Schritt II) bei Temperaturen von 30 bis 60°C erfolgt.

3. Verfahren gemäß Anspruch 1 oder 2, **dadurch gekennzeichnet, dass** die Abtrennung in Schritt III) bei Temperaturen von 0 bis < 30°C erfolgt.

4. Verfahren gemäß einem der Ansprüche 1 bis 3, **dadurch gekennzeichnet, dass** die Abtrennung in Schritt III) bei Temperaturen von 10 bis 25°C erfolgt.

5. Verfahren gemäß einem der Ansprüche 1 bis 4, **dadurch gekennzeichnet, dass** die Herstellung der Mischung in Schritt I) einstufig in einem Mischapparat erfolgt.

6. Verfahren gemäß einem der Ansprüche 1 bis 5, **dadurch gekennzeichnet, dass** die Verweilzeit in Schritt I) 1 Sekunde bis 2 Minuten beträgt.

7. Verfahren gemäß einem der Ansprüche 1 bis 6, **dadurch gekennzeichnet, dass** die Verweilzeit in Schritt I) 1 bis 20 Sekunden beträgt.

8. Verfahren gemäß einem der Ansprüche 1 bis 7, **dadurch gekennzeichnet, dass** das Volumenverhältnis von wässriger Glyoxal-Lösung zu lonentauscher-Lösung in Schritt I) 10:1 bis 1:3 beträgt.

9. Verfahren gemäß einem der Ansprüche 1 bis 8 , **dadurch gekennzeichnet, dass** die wässrige Glyoxal-Lösung mindestens eine Säure aus der Gruppe bestehend aus Ameisensäure, Essigsäure, Glyoxylsäure, Glykolsäure und Oxalsäure enthält.

## Claims

1. A process for purifying an aqueous glyoxal solution which comprises at least one acid by extractive acid removal, comprising the steps of
I) mixing an aqueous glyoxal solution with an ion exchanger solution comprising 20 to 60% by weight of a straight-chain or branched aliphatic tertiary amine with a molecular weight of from 300 to 600 g/mol and 80 to 40% by weight of one or more organic solvents with limited water miscibility, selected from aliphatic alcohols having from 3 to 15 carbon atoms, for a residence time of < 5 minutes and at a temperature of 30 to 100°C to prepare a mixture,
II) separating the mixture from step I) into an extracted aqueous glyoxal solution and a laden ion exchanger solution and removing the laden ion exchanger solution at temperatures of 30 to 100°C and
III) separating the extracted aqueous glyoxal solution into tertiary amine and purified aqueous glyoxal solution, and removing the tertiary amine at temperatures of < 30°C.

2. The process according to claim 1, wherein the separation in step II) is effected at temperatures of 30 to 60°C.

3. The process according to claim 1 or 2, wherein the removal in step III) is effected at temperatures of 0 to < 30°C.

4. The process according to any one of claims 1 to 3, wherein the removal in step III) is effected at temperatures of 10 to 25°C.

5. The process according to any one of claims 1 to 4, wherein the mixture is prepared in step I) in one stage in a mixing apparatus.

6. The process according to any one of claims 1 to 5, wherein the residence time in step I) is 1 second to 2 minutes.

7. The process according to any one of claims 1 to 6, wherein the residence time in step I) is 1 to 20 seconds.

8. The process according to any one of claims 1 to 7, wherein the volume ratio of aqueous glyoxal solution to ion exchanger solution in step I) is 10:1 to 1:3.

9. The process according to any one of claims 1 to 8, wherein the aqueous glyoxal solution comprises at least one acid from the group consisting of formic acid, acetic acid, glyoxylic acid, glycolic acid and oxalic acid.

## Revendications

1. Procédé pour la purification d'une solution aqueuse de glyoxal, qui contient au moins un acide, par séparation par extraction d'acide, comprenant les étapes
I) mélange d'une solution aqueuse de glyoxal avec une solution d'échangeur d'ions, contenant 20 à 60 % en poids d'une amine aliphatique tertiaire à chaîne droite ou ramifiée ayant une masse moléculaire de 300 à 600 g/mole et 80 à 40 % en poids d'un ou de plusieurs solvants organiques qui ne sont que partiellement miscibles à l'eau, choisis parmi des alcools aliphatiques ayant de 3 à 15 atomes de carbone, en un temps de séjour de < 5 minutes et à une température de 30 à 100 °C, pour l'obtention d'un mélange,
II) fractionnement du mélange provenant de l'étape I) en une solution aqueuse extraite de glyoxal et une solution d'échangeur d'ions chargée et séparation de la solution d'échangeur d'ions chargée, à des températures de 30 à 100 °C et
III) fractionnement de la solution aqueuse extraite de glyoxal en amine tertiaire et solution aqueuse de glyoxal purifiée et séparation de l'amine tertiaire à des températures < 30 °C.

2. Procédé selon la revendication 1, **caractérisé en ce que** le fractionnement dans l'étape II) s'effectue à des températures de 30 à 60 °C.

3. Procédé selon la revendication 1 ou 2, **caractérisé en ce que** la séparation dans l'étape III) s'effectue à des températures de 0 à < 30 °C.

4. Procédé selon l'une quelconque des revendications 1 à 3, **caractérisé en ce que** la séparation dans l'étape III) s'effectue à des températures de 10 à 25 °C.

5. Procédé selon l'une quelconque des revendications 1 à 4, **caractérisé en ce que** la préparation du mélange dans l'étape I) s'effectue en une étape dans un appareil de mélange.

6. Procédé selon l'une quelconque des revendications 1 à 5, **caractérisé en ce que** le temps de séjour dans l'étape I) va de 1 seconde à 2 minutes.

7. Procédé selon l'une quelconque des revendications 1 à 6, **caractérisé en ce que** le temps de séjour dans l'étape I) va de 1 à 20 secondes.

8. Procédé selon l'une quelconque des revendications 1 à 7, **caractérisé en ce que** le rapport en volume de la solution aqueuse de glyoxal à la solution d'échangeur d'ions dans l'étape I) vaut de 10:1 à 1:3.

9. Procédé selon l'une quelconque des revendications 1 à 8, **caractérisé en ce que** la solution aqueuse de glyoxal contient au moins un acide choisi dans le groupe constitué par l'acide formique, l'acide acétique, l'acide glyoxylique, l'acide glycolique et l'acide oxalique.
